# EUROPEAN PATENT SPECIFICATION

(11) **EP 3 562 463 B1**
(45) Date of publication and mention of the grant of the patent: **05.02.2025**
(21) Application number: 16826609.6
(22) Date of filing: 27.12.2016
(51) Int. Cl.: A61K 8/28, A61K 8/31, A61Q 15/00, A61K 8/92, A61K 8/02

(54) **EXTRUDED ANTIPERSPIRANT COMPOSITION FOR IMPROVED EFFICACY**
EXTRUDIERTE ANTIPERSPIRANT ZUSAMMENSETZUNG FÜR VERBESSERTE WIRKSAMKEIT
COMPOSITION ANTIPERSPIRANT EXTRUDÉE POUR UNE EFFICACITÉ AMÉLIORÉE

(43) Date of publication of application: 06.11.2019
(73) Proprietor: Colgate-Palmolive Company, New York, NY 10022 (US)
(72) Inventor: LINN, Elizabeth, Lyndhurst, NJ 07071 (US); MISNER, Steven, Verona, NJ 07044 (US); PAN, Long, Somerset, NJ 08873 (US); GROWE, JR., Ronald, Piscataway NJ 08854 (US)
(74) Representative: Wichmann, Hendrik
(86) International application number: PCT/US2016/068653
(87) International publication number: WO 2018/125030

(56) References cited:
- WO-A1-2011/040909
- WO-A1-2011/040911
- WO-A1-2011/050044
- WO-A1-2013/075906
- WO-A1-2014/088586
- WO-A1-97/16163
- WO-A2-2010/046011

## Description

### BACKGROUND

Antiperspirants are popular personal care products used to prevent or eliminate perspiration and body odor. The active agent responsible for this effect is usually an aluminum compound. The aluminum compound dissolves in the sweat or moisture on the skin surface of the armpit upon application of the antiperspirant. Upon dissolution, the aluminum compound acts to form a gel, which creates a small temporary 'plug' near the top of the sweat gland, significantly reducing the amount of sweat that is secreted to the skin surface.

While consumers look for antiperspirant products with favorable scents, easy to use packaging and quick drying formulations, the first and foremost in the hierarchy of consumer needs is long lasting control of odor and wetness. However, for many sensitive individuals, the high level of active agent in antiperspirants, often as much as 20 weight percent, can be an irritant to the skin. Reducing the amount of active agent to avoid this problem, however, can mitigate the efficacy of the antiperspirant.

Certain actives, such as aluminum zirconium tetrachlorohydrex glycine, are generally less likely to inflame the skin than other actives. However, these less inflammatory compounds may be costly and, consequently, antiperspirants containing high levels of these actives are expensive to manufacture. Accordingly, there remains a desire in the art for antiperspirant formulations that include reduced levels of actives, yet retain their efficacy and can be provided to consumers at a reasonable cost.
WO 2013/075906 A1 discloses an anhydrous soft solid composition comprising paraffin wax and hydrocarbon wax, wherein the weight ratio of the wax structurant to the carrier oil is from 0.085:1 to 0.2:1 and the carrier oil is present in an amount of at least 45% by weight.
WO 97/16163 A discloses a cosmetic stick deodorant composition comprising 0.5-10 wt% of an astringent metal salt active, a stable anhydrous base component comprising 5-40 wt% of a solidifying agent selected from the group consisting of high melting point waxes, low melting point wax fatty alcohols, fatty acid esters and fatty acid amides, 20-70 wt% of a volatile emollient, 10.50 wt% of non-volatile emollients and 0.05-10 wt% of a solubilizing agent having a hydrophile-liphophile balance value of greater than 10.
WO 2011/040909 A1 discloses a composition comprising at least one active chosen from an antiperspirant active and a deodorant active, a fatty acid in an amount of greater than 7 wt% and a plant oil in an amount of at least 12 wt%.
WO 2011/040911 A1 discloses a composition in which the volatile silicone is partially of fully replaced by a plant oil.
WO 2014/088586 A1 discloses a solid antiperspirant composition comprising at least one antiperspirant active, 2-15 wt% of a base component, wherein the base component is C8-C14 fatty acid in free acid form, and a gallant, wherein the gallant comprises less than 50 wt% of the gallant stearyl alcohol.
WO 2011/050044 A1 discloses an antiperspirant composition comprising a base, an antiperspirant active, and at least one antioxidant chosen from butylated hydroxytoluene, pentaerythrityl tetra-di-t-butyl hydroxyhydrocinnamate, caffeine, and abies picea extract in an amount that is greater than an amount for stabilizing the antiperspirant composition.
WO 2010/046011 A2 discloses a water-free antiperspirant stick comprising antiperspirant substances, low-melting waxes, and high-melting waxes.

### BRIEF SUMMARY

Provided herein are extruded antiperspirant compositions with improved efficacy. An extruded antiperspirant composition according to the invention is defined in claim 1. Preferred features are defined in the dependent claims. The following description describes embodiments useful for understanding the invention. Subjects treated with the present extruded antiperspirant compositions surprisingly exhibit a greater reduction in perspiration than those subjects treated with art-known antiperspirant formulations, including those that contain less active ingredient. In view of their excellent efficacy, yet decreased levels of antiperspirant active agent, the present extruded antiperspirant compositions are likely to be less costly than similarly efficacious formulations requiring higher levels of antiperspirant active agent.

More particularly, the present disclosure is directed to an extruded antiperspirant composition including: a) a solid component including: a solid structurant including at least one high melt wax having a melting temperature of at least 60° C, comprising at least one paraffin wax in a total amount of from 19 weight % to 29 weight %, a powder comprising an antiperspirant active agent, and b) a liquid component, wherein the solid component is present in the composition in an amount of at least 40 weight percent, wherein the solid structurant is present in the composition in an amount of at least 30 weight percent, wherein the liquid component is present in the composition in an amount of at least 35 weight percent, and wherein the antiperspirant active agent is present in the composition in an amount of 8-25 weight percent .

The high melt wax may have a melting point ranging from about 66° C to about 71° C.

In some implementations, the solid structurant includes at least one fatty acid in amounts as described herein, such as at least one C₁₆ to C₁₈ saturated fatty acid as described herein, e.g. palmitic acid, or any of the described triglycerides, such as hydrogenated soybean oil and palm stearin.

In some implementations, the powder component includes an inert filler as described herein, e.g., clay or a deodorant active material. In some implementations, talc is omitted from the powder component.

In some implementations, the antiperspirant active agent is, for example, an aluminum zirconim tetrachlorohydrex glycine complex or any of the antiperspirant active agents described herein. In some implementations, the antiperspirant active agent is present in the composition in an amount of about 15 weight % or less, such as about 14 weight % or in another amount as described herein.

In some implementations, the liquid component includes a plant oil, such as at least one of avocado, canola, corn, cottonseed, olive, palm, hi-oleic sunflower, mid-oleic sunflower, sunflower, palm kernel oil, coconut oil, safflower, babassu oils and combinations thereof. In other implementations, the liquid component includes a volatile silicone, such as caprylyl methicone. In other implementations, a volatile silicone is absent from the liquid component.

In some implementations, the liquid component further includes a semi-solid, e.g., a semi-solid occlusive agent, such as petrolatum.

In some implementations, an amount of solid component in the composition may be up to about 65% by weight of the composition while the liquid component is present in an amount of about 35 weight %. In some implementations, an amount of solid component in the composition may be present in an amount of up to about 60% by weight.

In some implementations, the extruded antiperspirant composition of the present disclosure reduces perspiration in a subject in a greater amount than does a comparative antiperspirant composition as described herein, e.g., one which is not an extrudate and/or, which does not contain a solid component in an amount of at least 40 weight percent, a solid structurant including at least one high melt wax as described herein in an amount of at least 30 weight percent and a liquid component in an amount of at least 35 weight percent.

In another aspect, the present disclosure is directed to a method including applying an extruded antiperspirant composition as described herein to an axillary area of a subject, such as a human subject.

### BRIEF DESCRIPTION OF THE DRAWINGS

The present disclosure will become more fully understood from the detailed description and the accompanying drawings, wherein:

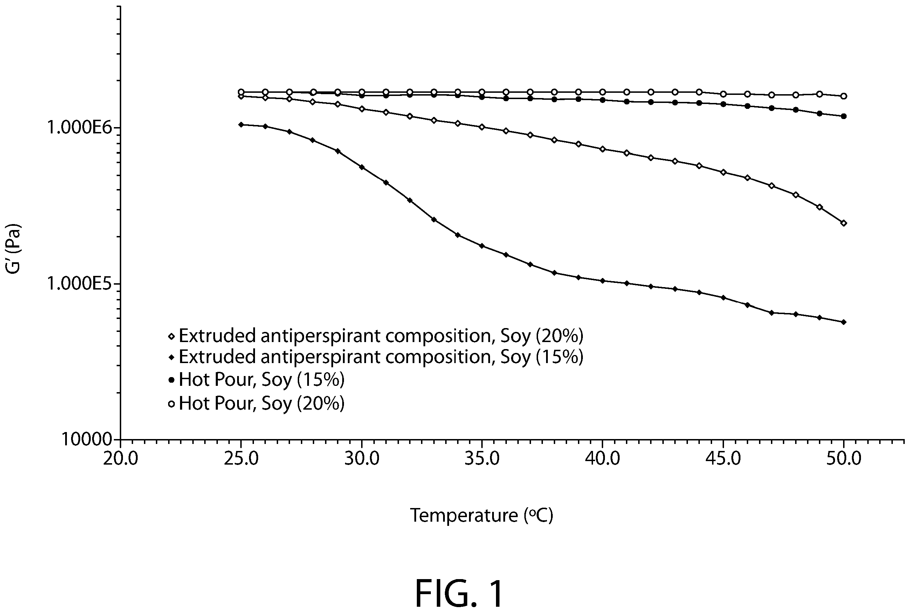

FIG. 1 depicts the elastic modulus G' (Pascals) as a function of temperature (°C) of extruded antiperspirant compositions according to the present disclosure and two conventional antiperspirant compositions as described in the Examples.

### DETAILED DESCRIPTION

The following description of various desired aspect(s) is merely exemplary in nature and is in no way intended to limit the invention, its application, or uses.

In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

The present disclosure is directed to an extruded composition. An "extruded composition" as used herein refers to a composition, which is formed by forcing a mixture of ingredients through an orifice resulting in an extrudate that is formed into a shape. In some implements, the mixture of ingredients is conveyed through one or more heating zones, typically by a screw mechanism. The screw or screws may be rotated by a variable speed motor inside a cylindrical barrel where only a small gap exists between the outside diameter of the screw and the inside diameter of the barrel. In this conformation, high shear is created at the barrel wall and between the screw elements by which the various components of a mixture are homogenized.

Typically, the extruded composition of the present disclosure is an antiperspirant or deodorant composition, more typically the extruded composition is an antiperspirant. As used herein, the term "extruded antiperspirant composition" refers to a coherent solid mass containing one or more antiperspirant active constituents. Typically, the extruded antiperspirant compositions are in the form of a solid stick, which is usually housed within a barrel container, typically of circular or elliptical cross section, that is open at one end through which the stick can protrude. However, other shapes, such as tablets, cakes or bars are also contemplated.

In some aspects, the extruded antiperspirant composition is anhydrous. As used herein, the term "anhydrous" refers to those antiperspirant implementations of the present disclosure that are substantially free of added water. From a formulation standpoint, this means that such implementations contain less than about 5%, more typically less than about 3%, even more typically less than about 1%, most typically zero percent, by weight of free or added water, other than the water of hydration typically associated with powder antiperspirant actives.

The extruded antiperspirant compositions of the present disclosure encompass those having a penetration force (product hardness) value of at least 600 gram-force, such as at least 750 gram-force, such as at least 800 gram-force, such as at least 1,400 gram-force, up to about 2,000 gram-force. Higher values represent harder product, and lower values represent softer product.

The term "product hardness" or "hardness" as used herein is a reflection of how much force is required to move a penetration cone a specified distance and at a controlled rate into the present extrudate antiperspirant compositions. These values are measured at 27° C., 15% relative humidity, using a TA-XT2 Texture Analyzer, available from Texture Technology Corp., Scarsdale, N.Y., U.S.A. The product hardness value as used herein represents the peak force required to move a standard 45° angle penetration cone through the composition for a distance of 5 mm at a rate of 1 mm/second.

### SOLID COMPONENT

### High Melt Wax Solid Structurants

The extruded antiperspirant composition of the present disclosure includes a solid component and a liquid component. The solid component typically includes at least one solid structurant and at least one powder component. As used herein, "a structurant" also known as a "gellant" refers to a material providing suspending, gelling, viscosifying, solidifying or thickening properties to a composition or which otherwise provides structure to a final product form. A "solid structurant" refers to a structurant material, which is, itself, a solid at ambient temperature (25° C).

The solid structurants of the present composition include a wax. The term "wax" as used herein is understood to mean a lipophilic fatty compound, which is solid at ambient temperature and which exhibits a reversible solid/liquid change of state. Typically, waxes have melting points ranging from about 30° C up to about 205° C, and, in the solid state, have an anisotropic crystalline arrangement.

Suitable waxes for use as solid structurants include high melting point waxes. As used herein, a "high melting point wax" refers to a wax having a melting temperature of at least 60° C, such as at least 65° C, at least 66 °C, at least 70° C or at least 80° C. For example, high melting point waxes useful in the present compositions may have a melting point from about 65° C to about 205° C, more typically from about 65° C to about 130° C and even more typically from about 65° C to about 104 ° C.

The high melting point wax may be selected, for example, from animal waxes, insect waxes, vegetable waxes, mineral waxes, petroleum waxes and synthetic waxes. Specific examples of waxes, without limitation, include carnauba, candelilla, castor, beeswax, spermaceti, microcrystalline, ozokerite, ceresin, polymethylene waxes and Fischer-Tropsch waxes. In some implementations, polyethylene wax may be used, such as PERFORMACOL^{®} 425 (C₂₀-C₄₀ alcohols and polyethylene) and PERFORMACOL^{®} 550 (C₃₀-C₅₀ alcohols and polyethylene), which are commercially available from, e.g., New Phase Technologies (Baker Hughes, Inc., Sugar Land, TX, U.S.A.).

The solid structurant comprises a high melt paraffin wax. As used herein, a "high melt paraffin wax" is a paraffin wax that has a melting temperature of at least 60° C. Typically, the high melt paraffin wax has a melting temperature ranging from about 66° C to about 85° C, such as about 66° C to about 71° C. High melt paraffin waxes are commercially available from, *e.g.*, Strahl & Pitsch Inc., West Babylon, NY U.S.A., Koster Keunen, LLC., Watertown, CT and Frank B. Ross Co., Rahway, NJ.

The total amount of the high melt paraffin waxes, are present in the extruded antiperspirant composition in an amount ranging from at 19 weight %, such as at least 20 weight %, such as at least 25 weight %, such as at least 27 weight %, such as at least 28 weight percent, such as at least 29 weight %, Typically, the total amount of high melting point wax ranges from about 19 weight percent to about 29 weight percent. In some implementations, the high melting point waxes are about 30 weight percent to about 40 weight %, such as about 30 weight %.

### Fatty acid structurants

In some implementations, the solid structurants of the present disclosure include agents in addition to the high melt waxes described herein. Such additional solid structurant agents include fatty acids, e.g., saturated fatty acids, esters of fatty acids, such as triglycerides or other cosmetically acceptable materials. Suitable saturated fatty acids include any C₁₆ to C₁₈ saturated fatty acid such as stearic acid and/or palmitic acid. Typically, the saturated fatty acid is palmitic acid. The amount of fatty acid in the composition may be greater than about 7% by weight up to 45 % by weight of the extruded antiperspirant composition. In other implementations, the amount of fatty acid may be at least 7% , 5% , 8% , 9%, 10%, 11%, 12%, 13%, 14%, 15%, 16%, 17%, 18%, 19%, 20%, 21%, 22%, 23%, 24%, 25%, 28%, 29%, 30% or 40% by weight. In certain implementations, the amount of saturated fatty acid is 15 to 21 weight %. In other implementations, the amount of saturated fatty acid is 16% to 20% by weight of the extruded antiperspirant composition.

Suitable triglyceride solid structurants are most typically obtained or derived from fully hydrogenated fats such as: (1) vegetable fats and oils including soybean, corn, sunflower, high erucic acid rapeseed, low erucic acid rapeseed, canola, crambe, meadowfoam, cottonseed, olive, safflower, sunflower and sesame seed,; (2) meat fats such as tallow or lard; (3) marine oils such as menhaden, pilcherd, sardine, whale or herring; (4) nut fats and oils such as coconut, palm or a fraction thereof such as palm stearin, palm kernel, babassu kernel, or peanut, Chinese Vegetable Tallow; (5) milkfat, butterfat; (6) cocoa butter and cocoa butter substitutes such as shea, or illipe butter; (7) structured triglycerides fats made from natural and synthetic routes; and (8) synthetic triglycerides made from hydrocarbon sources. Most typically, the triglycerides are palm stearin and/or hydrogenated vegetable oil, such as hydrogenated soybean oil or combinations thereof. The amount of trigylcerides in the composition may be greater than 0.001% up to about 40% by weight of the extruded antiperspirant composition, such as about 10 weight %, such as about 15 weight % to about 29 weight %, such as about 16 weight %, such as about 18 weight %, such as about 20 weight %, such as about 22 weight %, such as about 25 weight %, such as about 27 weight %. More typically, the amount of triglycerides in the composition ranges from about 10 weight % to about 20 weight %.

In some implementations, the amount of solid structurant present in the instant composition in total is at least 25 wt.%, such as at least 50 wt.%. In some implementations, the amount of solid structurant ranges from about 30 wt. % to about 40 wt. %, such as about 30 wt. %, such as about 34 wt. %, such as about 35 wt.%, such as about 40 wt. %, such as about 45 wt.% or such as about 46%. Typically, the amount of solid structurant in the present composition ranges from about 29 wt.% to about 45 wt.%. More typically, the amount of solid structurant in the present composition ranges from about 30 wt.% to about 40 wt. %.

### Powders

In some implementations, the solid component of the present disclosure includes a powder. As used herein, a "powder" refers to compositions or materials that are comprised of solid particles and are not dissolved in water or other solvents.

### Antiperspirant Actives

In some implementations, the powder of the present disclosure includes an antiperspirant active. Any of the known antiperspirant active materials can be utilized. Antiperspirant actives include, but are not limited to aluminum chlorhydrate, aluminum chloride, aluminum sesquichlorohydrate, aluminum-zirconium hydroxy chlorides, complexes or adducts of the above-mentioned active ingredients with glycol, such as propylene glycol (for example, "Rehydrol" II from Reheis Chemical Inc., Berkeley Heights, NJ) and combinations thereof. Known aluminum-zirconium salts in combination with neutral amino acids, such as glycine (e.g., aluminum-zirconium tetrachlorohydrex Gly) can also be used. Generally, any of the Category I active antiperspirant ingredients, listed in the Food and Drug Administration's Monograph on Antiperspirant Drug Products for over-the-counter human use (April 1, 2013) are suitable.

In other implementations, the antiperspirant active is an aluminum salt and/or an aluminum-zirconium salt, such as those described above, which are further stabilized by betaine and a calcium salt. More information about betaine and calcium salt stabilized antiperspirant salts can be found in U.S. Patent Application Publication No. 2006/0204463 to Tang et al.*.* In some embodiments, the betaine provides anti-irritation properties to the present extruded antiperspirant composition. In other embodiments, the present extruded antiperspirant composition exhibits fragrance stability upon aging when an aluminum-zirconium salt is used in association with betaine.

In other implementations, the antiperspirant active, such as those described above, is selected to have a low metal to chloride ratio. Examples of these antiperspirant actives can be found in U.S. Patent No. 6,375,937 to Chopra et al. and in U.S. Patent Application Publication No. 2004/0109833 to Tang et al.*.*

In other implementations, the antiperspirant active comprises a salt, such as an aluminum zirconium tetrasalt or an octasalt free of glycine, stabilized by betaine, which further has a metal to chloride ratio ranging from about 0.9:1 to about 1.3:1, such as from about 0.9:1 to about 1.2:1 or from about 0.9:l to about 1.1:1.

In some implementations, the antiperspirant active comprises an aluminum zirconium tetrasalt, wherein the Al/Zr atomic ratio ranges from about 3.2:1 to about 4.1:1.0 and the betaine:zirconium mole ratio ranges from about 0.2:1 to about 3.0:1, such as about 0.4:1 to about 1.5:1.

In some implementations, the antiperspirant active comprises aluminum zirconium octasalt free of glycine, wherein the Al/Zr atomic ratio ranges from about 6.2:1 to about 10.0:1 and the betaine:Zr mole ratio ranges from about 0.2:1 to about 3.0:1, such as about 0.4:1 to about 1.5:1. In the case of a salt that contains zirconium, the betaine may be incorporated during the synthesis of the salt so as to maximize the stabilizing effect this ingredient has (especially on the zirconium species). In other implementations, betaine can be added post synthesis to a glycine-free salt along with additional active phase ingredients to form a betaine stabilized active.

Examples of commercially available glycine-free tetrasalts and octasalts with a low metal to chloride ratio include REZAL^{™} AZP 955 CPG and REZAL^{™} AZP 885, respectively (both from Reheis Chemical Company, Berkeley Heights, NJ). A description of how such commercially available salts are prepared is found, for example, in U.S. Patent Nos. 7,074,394 and 6,960,338.

Methods for preparing antiperspirant actives containing aluminum, zirconium and glycine, which are stabilized with betaine, are described in United States Patent No. 7,105,691. Methods for preparing glycine-free antiperspirant salts stabilized with betaine are described in U.S. Patent Application Publication No. 2004/0198998.

.

Other suitable antiperspirant actives include aluminum nitratohydrate in combination with zirconyl hydroxychlorides and nitrates, aluminum-stannous chlorohydrates, an astringent salt of aluminum, an astringent salt of zirconium, aluminum bromohydrate, aluminum chlorohydrate, aluminum dichlorohydrate, aluminum sesquichlorohydrate, aluminum chlorohydrex PG, aluminum dichlorohydrex PG, aluminum sesquichlorohydrex PG, aluminum chlorohydrex PEG, aluminum dichlorohydrex PEG, aluminum sesquichlorohydrex PEG, aluminum chloride, aluminum sulfate, aluminum zirconium chlorohydrate, aluminum zirconium trichlorohydrate, aluminum zirconium tetrachlorohydrate, aluminum zirconium pentachlorohydrate, aluminum zirconium octachlorohydrate, aluminum zirconium tetrachlorhydrex propylene glycol, aluminum zirconium trichlorohydrex Gly, aluminum zirconium tetrachlorohydrex Gly, aluminum zirconium pentachlorohydrex Gly, aluminum zirconium octachlorohydrex Gly, buffered aluminum sulfate, potassium aluminum and sodium aluminum chlorohydroxy lactate. In some embodiments, the antiperspirant active is aluminum chlorhydrate. More typically, the antiperspirant active is an aluminum-zirconium glycine complex, such as an aluminum zirconium tetrachlorhydrex glycine complex.

Antiperspirant actives can be incorporated into the compositions according to the present disclosure in an amount of 8-25 weight %. Typically, about 15 to about 25 weight % of an antiperspirant active, such as about 18 weight % to about 25 weight %, such as about 19 weight % or such as about 20 weight % is used to achieve an efficacious formulation. In some embodiments, however, less than about 15 weight % of an antiperspirant active is needed to attain efficacy, such as about 14 weight % or such as about 8 weight % to about 13 weight %.

### Deodorant active materials

In some implementations, the powder component of the present composition includes a deodorant active material. Examples of suitable deodorant actives include 2,4,4'-trichloro-2'-hydroxy diphenyl ether (Triclosan), benzethonium chloride, polyhexamethylene biguanides and cetyl-trimethylammomium bromide.

### Inert filler materials

In some implementations, the powder component further includes inert filler materials, which may act as, e.g., a rheology modifier. Examples of suitable inert fillers include cornstarch, talc (magnesium silicate), fumed silica, clays, such as BENTONE^{®} (Elementis Specialties Inc., East Windsor, NJ) zeolites, a superabsorbent having little or no tack upon wetting, such as a superabsorbent polymer selected from the group consisting of starch graft homopolymers and copolymers of poly(2-propenamide-co-2-propenioic acid) sodium salt, such as WATERLOCK^{®} A 180 from Grain Processing Corp., Muscatine, Iowa, low melting point polyethylene (melting point ranging from about 45° to about 60°C) and mixtures thereof. In some implementations, talc is not included in the solid component. Typically, inert filler material is present in the composition of the disclosure in an amount ranging from about 2 wt. % to about 8 wt. %, such as about 4 wt. % to about 6 wt. %.

### Other solid components

In some implementations, the solid component of the present disclosure further comprises a solid emollient, a solid surfactant and/or other cosmetically suitable solid materials in any desired amount provided that the total amount of solids does not exceed about 80% by weight of the composition of the disclosure as described herein, more typically about 70 weight %, yet even more typically about 65 weight %, yet even more typically about 60 weight %. For example, in some implementations PEG-8 distearate is included in the solid component.

### LIQUID COMPONENT

The present solid antiperspirant compositions further contain a non-aqueous liquid component. As used herein, a "liquid" refers to those materials that are liquids at ambient temperature. In some implementations, the liquid component may further comprise a semi-solid component as described herein below.

Typically, the liquid component functions as a fluidizer. A "fluidizer" as used herein facilitates ease of blending and uniformity of the solid structurant and powder phases when combined, efficiently producing a homogenous blend of these formula components during processing. In some implementations, the liquids described herein for use in the present compositions may have multifunctional properties in terms of being a fluidizer and, in addition, functioning as a texturizer, an occlusive and/or an emollient.

As used herein, a "texturizer" is a material that provides the necessary product application aesthetics expected by the consumer, for example, soft/smooth feel e.g., as an antiperspirant stick is applied, good glide characteristics in use, etc. "Emollients" are a known class of materials in this art, imparting a soothing effect to the skin. Emollients help to maintain the soft, smooth, and pliable appearance of the skin. As used herein, an "occlusive agent" refers to an agent that increases moisture levels in the skin by providing a physical barrier to epidermal water loss.

Suitable liquid emollients for use in the liquid component of the present disclosure include fatty and/or aromatic carboxylic acid esters such as benzyl benzoate, isostearyl benzoate, C₁₂-C₁₅ alkyl benzoate, C₁₀-C₁₅ alkyl lactate, isopropyl myristate, isopropyl palmitate, propyl myristate having the general formula RCOOR', where R and R' may be the same or different, are from 2-20 carbon atoms, and may be saturated, unsaturated or aromatic; ethoxylated and/or propoxylated alcohols and acids such as PPG-14 myristyl ether, PPG-14 butyl ether, PPG-3 myristyl ether, myristeth-3 propionate, and similar materials known to those in the art; branched-chain hydrocarbons such as PERMETHYL^{®} (from Permethyl Corporation, Presperse, Inc., South Plainfield, NJ) and mixtures thereof. Typically, PPG-14 butyl ether is used. The liquid emollient is typically in the composition in an amount of at least 5%, such as about 7%, such as about 15% of the total weight of the composition. In some implementations, the liquid emollient may function as a fluidizer.

More typically, however, the present extruded antiperspirant compositions can include a volatile silicone. As used herein a "volatile material" means that the material, such as a volatile silicone, has a measurable vapor pressure at ambient temperature.

In some implementations, the volatile silicone spreads easily, and can function as a texturizer since it has a light "feel" and may be used to improve the spreadability of other materials in the present composition, e.g., plant oils. In some implementations, the volatile silicone may function as a fluidizer.

In certain implementations, the volatile silicone is a volatile cyclic polydimethylsiloxane (cyclomethicone), e.g., cyclopentasiloxane. Various types of cyclomethicones may be used. Illustratively, and not by way of limitation, the volatile silicones are one or more members selected from cyclic polydimethylsiloxanes, such as those represented by Formula I: 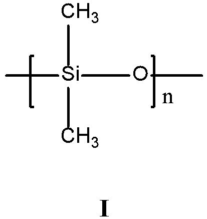 where n is an integer with a value of 3-7, particularly 5-6. Illustrative examples of suitable cyclomethicones are DC-345 and DC-245 fluids, manufactured by Dow Corning Corporation, Midland, MI.

In other implementations, the volatile silicone used in the liquid component of the present disclosure is caprylyl methicone, which is represented by Formula II: 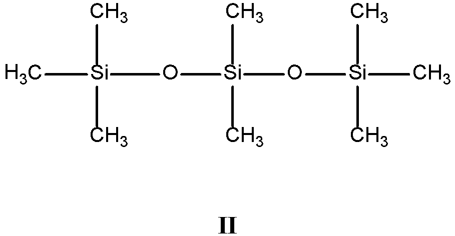 Caprylyl methicone is commercially available from, e.g., Dow Corning Corporation as F2-3196.

In one implementation, the amount of volatile silicone in the composition is greater than 0 up to 35 weight % of the composition. In another implementation, the amount is less than about 35 weight %, less than about 30 weight %, less than about 25 weight %, less than about 20 weight %, less than about 15 weight %, less than about 10 weight %, less than about 5 weight %, or less than about 1 weight % of the composition. In some implementations, there is no volatile silicone in the compositions of the disclosure.

In some implementations, the liquid component comprises a plant oil, which may function in the instant compositions as an emollient and/or fluidizer. The term "plant oil" refers to oil obtained from a plant, or made by blending oil components to obtain an oil that is substantially similar in composition to a plant oil. By "substantially similar", is meant that the manufactured oil contains at least 50 weight % (or at least 60, about 70, about 80, about 90, about 95, about 98, or about 99 weight %) of the components that are found in the plant oil that it is designed to mimic.

Examples of plant oils that may be used in the liquid component of the present disclosure include, but are not limited to, palm kernel, avocado, canola, corn, cottonseed, olive, palm, hi-oleic sunflower, mid-oleic sunflower, sunflower, palm kernel olein, safflower, babassu oils, coconut oil and combinations thereof. Typically, palm kernel oil is the selected oil.

In certain implementations, the plant oil contains up to 40 weight % C₁₂-C₁₄ fatty acids, *e.g.,* palm kernel oil. These oils may provide stick antiperspirant products with greater strength than oils that do not contain at least 40 weight % C₁₂-C₁₄ fatty acids.

In other implementations, the plant oil has a low amount of unsaturation. Higher levels of unsaturation could result in undesired fragrance when the unsaturated bonds become saturated over time. In certain implementations, the amount of unsaturated components in the oil is no more than about 20, about 19, about 18, about 17, about 16, about 15, about 14, about 13, about 12, about 11, or about 10 weight % of the oil. Representative compositions of selected oils are found, for example, in U.S. Publication Number 2011/0076310,.

The amount of plant oil in the present composition is at least 8 weight %, at least 9 weight percent or at least 12 weight % of the composition. In certain implementations, the amount is about 18, about 20, about 28, about 30 or about 35 weight % in the composition. In some implementations, the amount of plant oil is greater than the amount of volatile silicone in the composition. In one implementation, there is no volatile silicone in the composition. In other implementations, the amount of plant oil is more than about 10, about 15, about 20, about 25, about 30, about 35, about 40, about 45, about 50, about 55, about 60, about 65, about 70, about 75, about 80, about 85, about 90, or about 95 weight % of the combined weight of plant oil and volatile silicone (if present). In another implementation, the combined amount of the plant oil and volatile silicone is up to about 35 weight %.

### Semi-Solids

In some implementations, the liquid component further comprises an Ingredient that is semi-solid at ambient temperature. As used herein, the terms "semi-solid" and "liquids" refer to fluids distinguished on the basis of viscosity: a semi-solid is a high viscosity fluid, while a liquid has lower viscosity. There is no definitive dividing line between these two types of fluids. A semi-solid can, in certain embodiments, have a viscosity as high as thousands of mPa·s. Semi-solids may include pastes, ointments and gels.

In some implementations, the semi-solid is an occlusive agent. A typical nonlimiting semi-solid occlusive agent for use in the present extruded antiperspirant composition is petrolatum. The occlusive agent may be in any desired amount provided that the total amount of liquids and semi-solids does not exceed the amount of the solid component, such as about 35 to about 40 weight percent liquid component.

As indicated herein, the present extruded antiperspirant compositions include a solid component, a liquid component and, optionally, a semi-solid component. Typically, the total amount of solid component is greater than the total amount of liquid component. In other implementations, the solid component is greater than the total amount of liquid component in combination with the semi-solids. More typically, the total amount of solids does not exceed about 70 wt. %, such as about 40 wt. % to about 65 wt. %, such as about 45 wt. %, about 50 wt. % or about 55 wt. %. Even more typically, the total amount of solids does not exceed 60 wt. %. In some implementations, the total amount of liquid component or liquid/semi-solid component ranges from about 30 wt. % to about 45 wt %, such as about 30 wt. % to about 37 wt. %, such as about 35 wt. % or such as about 40 wt%.

In some implementations, the extruded antiperspirant composition of the present disclosure is more efficacious than other antiperspirant compositions that are not extrudates and/or which do not contain the same ingredients and/or which do not contain a solid component, including the structurant component, and a liquid component in the amounts described herein. For example, in some implementations, the extruded antiperspirant composition of the present disclosure reduces perspiration in a subject, such as human subject, in a greater amount than that of a comparative antiperspirant composition, which does not contain a solid component in an amount of at least 40 weight percent, a solid structurant including at least one high melt wax in an amount of at least 30 weight percent and a liquid component in an amount of at least 35 weight percent. In some implementations, the extruded antiperspirant composition of the present disclosure is more efficacious than a comparative antiperspirant composition that contains less active ingredient than that of the extruded antiperspirant composition of the present disclosure.

### METHODS OF PREPARATION

*et al.,.* Briefly, U.S. Patent No. 7,128,901 discloses a method comprising mixing and combining the agents of the solid structurants into a well-mixed blend, homogenizing the structurant mixture, and then mixing the homogenized solid mass with the liquid and powder components. The resultant mixture containing the structurant, liquid and powder is then homogenized. The homogenate is subsequently extruded to obtain a uniform, solid, cohesive extrudate. The extrudate may then be cut into billets of a selected length, after which unit products are formed, e.g., antiperspirant sticks.

Mixing may be effected by, e.g., a Sigma mixer (Paresh Engineering Co., Mumbai), an Agi mixture, a Ribbon mixer, or any other mixer that is capable of handling sticky ingredients and that can work powders and liquids into a relatively homogeneous mass. Homogenizaton may be performed by milling using a roll mill or with a twin screw extruder, a single screw extruder, or extruders of both types with or without screens and with or without perforated plates.

The extrudates may be cut to any desired length to form billets. The billets may be used as is or subjected to further steps. For example, the billets may be subjected to a thermal environment that is sufficient to create a smoother surface, or subjected to microwave treatment that is sufficient to smooth the surface and/or modify the texture of the billet. The final form of the unit can be packaged as is in a conventional plastic container or wrapped for consumer use.

### EXAMPLES

### Example 1: Prototype Formulations

A variety of prototype formulations were prepared and assessed for their ability to reduce perspiration in a subject. Each of the prototype formulations contained varying amounts of solid and liquid components. The solid components included varying amounts of high melt wax solid structurants and fatty acid solid structurants (high melt paraffin wax, hydrogenated soybean oil and optionally, palm stearin) or fatty acid solid structurants only (stearic acid). Additional solid component ingredients included a surfactant (PEG-8 distearate) and powders including antiperspirant active agent (Aluminum Zirconim Tetrachlorohydrex Glycine Complex) and optionally, talc. In order to determine the minimum amount of antiperspirant active agent that may be needed to reduce perspiration in a subject, the prototype formulations incorporated varying amounts of Aluminum Zirconim Tetrachlorohydrex Glycine Complex, ranging from 8 weight percent to 20 weight percent.

The liquid components of the prototype formulations included emollients, such as plant oils (palm kernel oil) and PPG 14 Butyl Ether. A semi-solid occlusive (petrolatum) was optionally included. Examples of prototype formulations, which were used to determine the types and amounts of ingredients included in the extruded antiperspirant compositions of the presented disclosure are provided in Table 1, below. All ingredients in Table 1 are expressed in weight % relative to the total weight of the formulation.

**Table 1. Examples of Prototype Formulations**

| Ingredient | 1 | 2 | 3 | 4 | 5 | 6 | 7 | 8 |
|---|---|---|---|---|---|---|---|---|
| **Aluminum Zirconim Tetrachlorohydrex Glycine Complex** | 14.00 | 14.00 | 14.00 | 14.00 | 10.00 | 20.00 | 8.00 | 14.00 |
| **Palm Kernel Oil** | 30.18 | 36.80 | 18.40 | 27.43 | 30.18 | 15.81 | 9.60 | 16.44 |
| **PPG 14 Butyl Ether** | 7.54 | 9.20 | 9.20 | 7.54 | 7.54 | 3.95 | 10.33 | 10.33 |
| **Paraffin** | 28.78 | 19.50 | 19.50 | 28.78 | 28.78 | 0.00 | 0.00 | 0.00 |
| **Stearic Acid** | 0.00 | 0.00 | 0.00 | 0.00 | 0.00 | 36.56 | 26.24 | 26.24 |
| **Hydrogenated Soybean Oil** | 15.50 | 10.50 | 10.50 | 15.50 | 15.50 | 19.68 | 26.24 | 26.24 |
| **PEG 8 Distearate** | 4.00 | 4.00 | 4.00 | 4.00 | 4.00 | 4.00 | 4.00 | 4.00 |
| **Talc** | 0.00 | 6.00 | 6.00 | 0.00 | 4.00 | 0.00 | 6.00 | 0.00 |
| **Petrolatum** | 0.00 | 0.00 | 0.00 | 2.75 | 0.00 | 0.00 | 0.00 | 2.75 |
| **Palm Stearin** | 0.00 | 0.00 | 18.40 | 0.00 | 0.00 | 0.00 | 9.59 | 0.00 |
| **Total** | 100.00 | 100.00 | 100.00 | 100.00 | 100.00 | 100.00 | 100.00 | 100.00 |

Solid antiperspirant compositions were prepared from each of the prototype formulations by either 1) mixing the solid and liquid components, followed by extrusion to form a stick product or 2) by using a "hot pour" process as is known in the art. Briefly, in a hot pour process, the total liquid component, which is in amount ranging from about 40 wt. % to about 50 wt. %, and the solid structurant, which is in an amount of about 20 wt.% total, are combined and heated. The powder components, e.g., antiperspirant active agents, are then combined with the melted components and mixed. After mixing, the composition is poured into a stick-form mold (for example, dispensing containers), as is conventional in the art, after which the compositions harden into a solid.

The prototype solid antiperspirant compositions described in this Example as well as other prototype solid antiperspirant compositions according to the present disclosure were evaluated to assess, e.g., efficacy, rheological characteristics, etc. The results of these studies are described below.

### Example 2: Evaluation of sweat reduction

Efficacy of extruded antiperspirant sticks, which were prepared using high melt paraffin wax and soybean hydrogenated oil as the solid structurants according to the present disclosure, were evaluated via a sweat reduction protocol. Efficacy of the instant extruded antiperspirant sticks was compared to that of prototypes prepared with stearic acid structurants and prototype antiperspirant sticks, prepared by the hot pour method.

Efficacy was assessed on thirty-six volunteers using a hot room protocol. Initially, baseline sweat collection values were obtained by affixing absorbent pads on the backs of volunteers, subjecting them to a hot room and then determining the amount of sweat absorbed by the pads gravimetrically. The next day, test products were applied to the backs of the volunteers, covered with gauze and taped. The test products included ((i) three extruded antiperspirant sticks containing high melt paraffin wax and 14% antiperspirant active agent according to the present disclosure (ii) two extruded antiperspirant sticks containing stearic acid and 20% or 14% antiperspirant active agent and (iii) four antiperspirant sticks containing hydrogenated soy oil, palm kernel oil and antiperspirant active agents in amounts ranging from 5% to 15%, prepared by the hot pour method ("Hot Pour Soy/PKO")). Two control antiperspirant sticks (commercially available high and medium efficacy antiperspirant sticks) were also applied to the volunteers' backs. Untreated sites, which served as controls, were covered with gauze and taped similarly to the test and control product sites. After twenty-four hours, the gauze was removed, the sites were washed and the test and control products re-applied. For two more consecutive days, the test and control products were again applied onto the volunteers' backs in the manner described above.

Twenty four hours after the third application, absorbent pads were placed on the backs of the volunteers at the location where the test and control products were previously applied. An absorbent pad was also applied to the untreated control site. After again subjecting the volunteers to the hot room, the absorbent pads at the treated control, the untreated control and test sites were removed and the amount of sweat absorbed by the pads at the control and test sites was gravimetrically determined.

As expected, the high efficacy control antiperspirant stick resulted in greater sweat reduction (Sample J, 53.1%) than that of the medium efficacy control antiperspirant stick (Sample K, 31.2%). *See* Table 2. Unexpectedly, however, the extruded antiperspirant sticks of the present disclosure, which included high melt paraffin wax as a solid structurant, performed better than the medium efficacy control antiperspirant sticks. Further, the extruded antiperspirant sticks of the present disclosure also demonstrated greater sweat reduction than the extruded antiperspirant sticks that contained only stearic acid as a solid structurant.

In particular, the percentage of sweat reduction observed for the three extruded antiperspirant high melt wax paraffin-containing sticks of the present disclosure was 50.7% (Sample C in Table 2), 56.8% (Sample A in Table 2) and 60.1% (Sample B in Table 2). This percentage in sweat reduction was significantly greater than the 31.2% sweat reduction observed for the medium efficacy control (p<0.5). The results also revealed that the addition of talc to the high melt paraffin-containing extruded antiperspirant stick formulation did not enhance the percentage of sweat reduction (50.7% (Sample C, sweat reduction with talc) versus 56.8% (Sample A, without talc) and 60.1% (Sample B, without talc).

In contrast to the high percentage of sweat reduction observed at the sites treated with extruded antiperspirant sticks containing high melt paraffin in accordance with the instant disclosure, the extruded antiperspirant sticks that only contained a stearic acid solid structurant, performed significantly worse than the medium efficacy control. As shown in Table 2, the percentage of sweat reduction observed for two stearic acid-containing extruded antiperspirant sticks was only 18.9% (Sample D) and 6.7% (Sample E). This percentage in sweat reduction was significantly less than the 31.2% sweat reduction observed for the medium efficacy control (p<0.5).

Antiperspirant sticks prepared by the hot pour method also demonstrated poor sweat reduction in comparison to that of the extruded antiperspirant sticks of the present disclosure. As evident in Table 2, only the hot pour stick containing 15% antiperspirant active agent performed at parity with the medium control (31.4% vs. 31.2%). All other Hot Pour Soy/PKO antiperspirant sticks performed significantly worse than the medium efficacy control (p <0.05). See Table 2, Sample I (18.5% sweat reduction, 10% active antiperspirant agent), Sample H (16.7% sweat reduction, 8% active antiperspirant agent) and Sample G (+1.7 percent sweat increase, 5% active antiperspirant agent). Accordingly, the extruded antiperspirant sticks of the present disclosure resulted in superior efficacy in comparison to Hot Pour Soy/PKO antiperspirant sticks. This superior efficacy was achieved even when the instant extruded antiperspirant sticks included less antiperspirant active agent than that of the Hot Pour Soy/PKO antiperspirant sticks. See Samples A-C of Table 2, which contained only 14% antiperspirant active agent, but which exhibited a percent sweat reduction ranging from 50.7% to 60.1% in comparison to the Hot Pour Soy/PKO Sample F, which contained 15% antiperspirant active agent, but only exhibited 31.4% sweat reduction.

**Table 2**

| Sample | Antiperspirant | % sweat reduction vs. untreated site |
|---|---|---|
| B* | Extruded Stick Paraffin | 60.1 |
| A* | Extruded Stick Paraffin | 56.8 |
| C* | Extruded Stick Paraffin with Talc | 50.7 |
| D* | Extruded Stick Stearic Acid | 18.9 |
| E* | Extruded Stick Stearic Acid | 6.7 |
| K | Medium Efficacy Control | 31.2 |
| J* | High Efficacy Control | 53.1 |
| F | Hot Pour Soy/PKO 15% Active | 31.4 |
| I* | Hot Pour Soy/PKO 10% Active | 18.5 |
| H* | Hot Pour Soy/PKO 8% Active | 16.7 |
| G* | Hot Pour Soy/PKO 5% Active | +1.7 |

| | | |
|---|---|---|
| *significantly different from Medium Efficacy Control p<0.05 | | |

### Example 3. Rheology

The rheology profiles for two extruded antiperspirant sticks containing high melt paraffin wax and 15 wt. % hydrogenated soy oil or 20 wt. % hydrogenated soy oil according to the present disclosure and two Hot Pour Soy/PKO antiperspirant sticks containing 15% hydrogenated soy oil or 20% hydrogenated soy oil were assessed by determining the elastic modulus (G'). The elastic modulus represents the degree of structure in a sample. G' was determined at ambient temperature for the antiperspirant sticks using a Stress Rheometer, available from TA Instruments, New Castle, Delaware]. Sample sizes of about 5 grams of the instant extruded antiperspirant sticks or the Hot Pour Soy/PKO antiperspirant sticks were obtained and placed between the plate fixtures on the rheometer for measurements of G' in Pascals.

The results are depicted in FIG. 1. As shown in FIG. 1, the extruded antiperspirant sticks according to the present disclosure have a unique rheology profile in comparison to that of the antiperspirant sticks prepared according to the hot pour method. In particular, the present extruded antiperspirant sticks demonstrate a decrease in G' much more rapidly as the temperature is increased between 30°C and 50°C than the decrease in G' observed for the antiperspirant sticks prepared by the hot pour process. Without being bound by theory, it is contemplated that the unique rheology of the present extruded antiperspirant compositions may contribute to their superior efficacy as shown in Example 2.

## Claims

1. An antiperspirant composition obtainable by extrusion of a composition comprising:
a) a solid component comprising:
a solid structurant comprising at least one high melt wax having a melting temperature of at least 60 °C, comprising at least one paraffin wax having a melting temperature of at least 60° C in a total amount of from 19 weight percent to 29 weight percent in the antiperspirant composition,
a powder comprising an antiperspirant active agent, and
b) a liquid component,
wherein the solid component is present in the composition in an amount of at least 40 weight percent,
wherein the solid structurant is present in the composition in an amount of at least 30 weight percent,
wherein the liquid component is present in the composition in an amount of at least 35 weight percent,
wherein the antiperspirant active agent is present in the composition in an amount of 8 to 25 weight percent.

2. The extruded antiperspirant composition of claim 1, wherein the solid structurant is present in the composition in an amount ranging from 30 weight % to 40 weight %.

3. The extruded antiperspirant composition of any of the preceding claims, wherein the solid structurant further comprises at least one fatty acid selected from the group consisting of a C₁₆ to C₁₈ saturated fatty acid and a triglyceride.

4. The extruded antiperspirant composition of claim 3, wherein the triglyceride comprises at least one compound selected from the group consisting of hydrogenated soybean oil and palm stearin.

5. The extruded antiperspirant composition of any of the preceding claims, wherein the liquid component comprises a plant oil.

6. The extruded antiperspirant composition of claim 5, wherein the plant oil comprises at least one oil selected from the group consisting of avocado, canola, corn, coconut, cottonseed, olive, palm, hi-oleic sunflower, mid-oleic sunflower, sunflower, palm kernel oil, safflower, and babassu oils.

7. The extruded antiperspirant composition of claim 1, wherein the antiperspirant active agent is an aluminum zirconium tetrachlorohydrex glycine complex and wherein the aluminum zirconium tetrachlorohydrex glycine complex is present in the composition in an amount less than 15 weight percent.

8. The extruded antiperspirant composition of any of the preceding claims , wherein the liquid component comprises caprylyl methicone.

9. The extruded antiperspirant composition of any of the preceding claims, wherein the solid component is present in an amount of up to 60% by weight of the composition.

## Patentansprüche

1. Schweißhemmende Zusammensetzung, erhältlich durch Extrusion einer Zusammensetzung umfassend:
a) eine feste Komponente, umfassend:
ein festes Strukturierungsmittel, umfassend mindestens ein hochschmelzendes Wachs mit einer Schmelztemperatur von mindestens 60°C, umfassend mindestens ein Paraffinwachs mit einer Schmelztemperatur von mindestens 60°C in einer Gesamtmenge von 19 Gewichtsprozent bis 29 Gewichtsprozent in der schweißhemmenden Zusammensetzung,
ein Pulver, das einen schweißhemmenden Wirkstoff umfasst, und
b) eine flüssige Komponente,
wobei die feste Komponente in der Zusammensetzung in einer Menge von mindestens 40 Gewichtsprozent vorhanden ist,
wobei das feste Strukturierungsmittel in der Zusammensetzung in einer Menge von mindestens 30 Gewichtsprozent vorhanden ist,
wobei die flüssige Komponente in der Zusammensetzung in einer Menge von mindestens 35 Gewichtsprozent vorhanden ist,
wobei der schweißhemmende Wirkstoff in der Zusammensetzung in einer Menge von 8 bis 25 Gewichtsprozent vorhanden ist.

2. Extrudierte schweißhemmende Zusammensetzung nach Anspruch 1, wobei das feste Strukturierungsmittel in der Zusammensetzung in einer Menge von 30 Gewichtsprozent 40 Gewichtsprozent vorhanden ist.

3. Extrudierte schweißhemmende Zusammensetzung nach einem beliebigen der vorhergehenden Ansprüche, wobei das feste Strukturierungsmittel weiterhin mindestens eine Fettsäure umfasst, die ausgewählt ist aus der Gruppe bestehend aus einer gesättigten C₁₆ bis C₁₈ Fettsäure und einem Triglycerid.

4. Extrudierte schweißhemmende Zusammensetzung nach Anspruch 3, wobei das Triglycerid mindestens eine Verbindung umfasst, die ausgewählt ist aus der Gruppe bestehend aus hydriertem Sojabohnenöl und Palmstearin.

5. Extrudierte schweißhemmende Zusammensetzung nach einem beliebigen der vorhergehenden Ansprüche, wobei die flüssige Komponente ein Pflanzenöl umfasst.

6. Extrudierte schweißhemmende Zusammensetzung nach Anspruch 5, wobei das Pflanzenöl mindestens ein Öl umfasst, das ausgewählt ist aus der Gruppe bestehend aus Avocado-, Canola-, Mais-, Kokosnuss-, Baumwollsamen-, Oliven-, Palmen-, hoch-oleinhaltigen Sonnenblumen-, mittel-oleinhaltigen Sonnenblumen-, Sonnenblumen-, Palmkernöl-, Saflor- und Babassu-Ölen.

7. Extrudierte schweißhemmende Zusammensetzung nach Anspruch 1, wobei der schweißhemmende Wirkstoff ein Aluminium-Zirkonium-Tetrachlorhydrex-Glycin-Komplex ist und wobei der Aluminium-Zirkonium-Tetrachlorhydrex-Glycin-Komplex in der Zusammensetzung in einer Menge von weniger als 15 Gewichtsprozentvorhanden ist.

8. Extrudierte schweißhemmende Zusammensetzung nach einem beliebigen der vorhergehenden Ansprüche, wobei die flüssige Komponente Caprylylmethicon umfasst.

9. Extrudierte schweißhemmende Zusammensetzung nach einem beliebigen der vorhergehenden Ansprüche, wobei die feste Komponente in einer Menge von bis zu 60 Gewichtsprozent der Zusammensetzung vorhanden ist.

## Revendications

1. Composition anti-transpirante pouvant être obtenue par extrusion d'une composition comprenant :
a) un constituant solide comprenant :
un agent structurant solide comprenant au moins une cire à point de fusion élevé ayant une température de fusion d'au moins 60 °C, comprenant au moins une cire de paraffine ayant une température de fusion d'au moins 60 °C en une quantité totale de 19 pour cent en poids à 29 pour cent en poids dans la composition anti-transpirante,
une poudre comprenant un agent actif anti-transpirant, et
b) un constituant liquide,
dans laquelle le constituant solide est présent dans la composition en une quantité d'au moins 40 pour cent en poids,
dans laquelle l'agent structurant solide est présent dans la composition en une quantité d'au moins 30 pour cent en poids,
dans laquelle le constituant liquide est présent dans la composition en une quantité d'au moins 35 pour cent en poids,
dans laquelle l'agent actif anti-transpirant est présent dans la composition en une quantité de 8 à 25 pour cent en poids.

2. Composition anti-transpirante extrudée selon la revendication 1, dans laquelle l'agent structurant solide est présent dans la composition en une quantité allant de 30 % en poids à 40 % en poids.

3. Composition anti-transpirante extrudée selon l'une quelconque des revendications précédentes, dans laquelle l'agent structurant solide comprend en outre au moins un acide gras choisi dans le groupe constitué par un acide gras saturé en C₁₆ à C₁₈ et un triglycéride.

4. Composition anti-transpirante extrudée selon la revendication 3, dans laquelle le triglycéride comprend au moins un composé choisi dans le groupe constitué par l'huile de soja hydrogénée et la stéarine de palme.

5. Composition anti-transpirante extrudée selon l'une quelconque des revendications précédentes, dans laquelle le constituant liquide comprend une huile végétale.

6. Composition anti-transpirante extrudée selon la revendication 5, dans laquelle l'huile végétale comprend au moins une huile choisie dans le groupe constitué par les huiles d'avocat, de canola, de maïs, de noix de coco, de graines de coton, d'olive, de palme, de tournesol à haute teneur en acide oléique, de tournesol à teneur moyenne en acide oléique, de tournesol, l'huile de palmiste, les huiles de carthame et de babassu.

7. Composition anti-transpirante extrudée selon la revendication 1, dans laquelle l'agent actif anti-transpirant est un complexe aluminium zirconium tétrachlorohydrex glycine et dans laquelle le complexe aluminium zirconium tétrachlorohydrex glycine est présent dans la composition en une quantité inférieure à 15 pour cent en poids.

8. Composition anti-transpirante extrudée selon l'une quelconque des revendications précédentes, dans laquelle le constituant liquide comprend de la caprylyl méthicone.

9. Composition anti-transpirante extrudée selon l'une quelconque des revendications précédentes, dans laquelle le constituant solide est présent en une quantité allant jusqu'à 60 % en poids de la composition.
